(19) Europäisches Patentamt
European Patent Office
Office européen des brevets

(11) Veröffentlichungsnummer : **0 109 623**
**B1**

(12) # EUROPÄISCHE PATENTSCHRIFT

(45) Veröffentlichungstag der Patentschrift : **09.10.85**

(21) Anmeldenummer : **83111276.8**

(22) Anmeldetag : **11.11.83**

(51) Int. Cl.⁴ : **C 07 C 93/00**, C 07 C149/00, C 07 D295/08, C 07 C125/065, A 61 K 31/135// C07C47/00, C07D295/10

(54) **Phenylpropanolamine, ihre Herstellung und Verwendung.**

(30) Priorität : **20.11.82 DE 3242922**

(43) Veröffentlichungstag der Anmeldung : **30.05.84 Patentblatt 84/22**

(45) Bekanntmachung des Hinweises auf die Patenterteilung : **09.10.85 Patentblatt 85/41**

(84) Benannte Vertragsstaaten : **AT BE CH DE FR GB IT LI NL SE**

(56) Entgegenhaltungen : **FR-A- 2 034 453**

(73) Patentinhaber : **BASF Aktiengesellschaft**
**Carl-Bosch-Strasse 38**
**D-6700 Ludwigshafen (DE)**

(72) Erfinder : **Treiber, Hans Joerg, Dr.**
**Sperberweg 1**
**D-6831 Bruehl (DE)**
Erfinder : **Hofmann, Hans Peter, Dr.**
**Untere Hart 12**
**D-6703 Limburgerhof (DE)**
Erfinder : **Kreiskott, Horst, Prof. Dr.**
**Am Boehlig**
**D-6706 Wachenheim (DE)**
Erfinder : **Teschendorf, Hans-Jürgen, Dr.**
**Georg-Nuss-Strasse 5**
**D-6724 Dudenhofen (DE)**
Erfinder : **Traut, Martin, Dr.**
**Muehltalstrasse 125**
**D-6900 Heidelberg (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 109 623 B1

Jouve, 18, rue St-Denis, 75001 Paris, France

**Beschreibung**

Die Erfindung betrifft neue Phenylpropanolamine, Verfahren zu deren Herstellung und deren Verwendung bei der Bekämpfung von Krankheiten.

Die Phenoxypropanolamin-Teilstruktur ist ein wesentliches Strukturmerkmal bekannter Betarezeptorenblocker, wie Toliprolol oder Bupranolol. Weiter sind aus der US-A-2 695 919 phenoxysubstituierte Phenylalkanolamine bekannt, die lokalanaesthetische und bronchodilatatorische Wirkungen besitzen. Schließlich sind in der FR-A-2 034 453 [3-Alkoxy-bzw. 3-Aryloxy-2-(diaryl-hydroxy)-methyl]-propylamine beschrieben, die auf den Herzmuskel wirden, das ZNS beeinflussen sowie das Wachstum von Mikroorganismen hemmen.

Es wurden nun strukturell veränderte Verbindungen mit einem anderen Wirkungsspektrum gefunden.

Gegenstand der Erfindung sind Phenylpropanolamine der Formel I

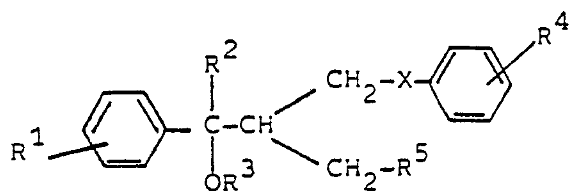

(I)

worin

$R_1$ ein Wasserstoff- oder ein Halogenatom,

$R^2$ ein Wasserstoffatom, einen gesättigtten oder ungesättigten Kohlenwasserstoffrest mit bis zu 15 Kohlenstoffatomen

$R^3$ ein Wasserstoffatom, einen gesättigten oder ungesättigten Kohlenwasserstoffrest mit bis zu 3 Kohlenstoffatomen, eine $C_{1-4}$-Acyl-, Benzoyl-, $C_{2-4}$-Carbalkoxygruppe oder eine Carbonamidogruppe -CO-NHR$^6$, worin $R^6$ eine $C_{1-4}$-Alkylgruppe darstellt,

$R^4$ ein Wasserstoff- oder Halogenatom,

$R^5$ eine Dimethylamino-, Piperidino- oder Pyrrolidinogruppe und

X ein Sauerstoff- oder Schwefelatom

bedeuten, sowie deren Salze mit physiologisch veträglichen Säuren.

In der Formel I bedeuten $R^1$ und $R^4$ vorzugsweise Fluor-, Chlor- oder Brom- atome in der para-Stellung, insbesondere aber Wasserstoffatome. $R^2$ ist vorzugsweise ein Wasserstoffatom oder eine $C_{1-4}$-Alkylgruppe und $R^3$ vorzugsweise ein Wasserstoffatom oder eine $C_{1-2}$-Acylgruppe. $R^5$ ist vorzugsweise eine Dimethylaminogruppe.

Die Phenylpropanolamine der Formel I lassen sich herstellen, indem man eine Verbindung der Formel II

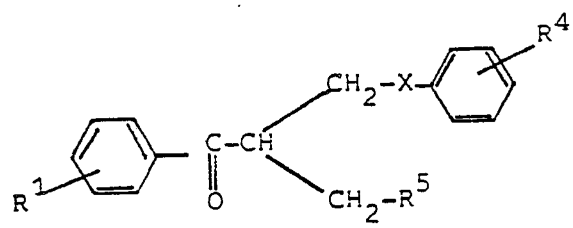

(II)

worin $R^1$, $R^4$, $R^5$ und X die vorstehend genannte Bedeutung haben,

a) reduziert oder

b) mit einer entsprechend substituierten metallorganischen Verbindung umsetzt

und in die so erhaltenen Verbindungen gegebenenfalls verethert oder verestert und/oder in ihre Salze mit physiologisch verträglichen Säuren überführt.

Die Reduktion a) der Verbindungen II wird in bekannter Weise mit katalytisch erregtem Wasserstoff, wobei beispielsweise Palladium-Kohle-Katalysator in einem Alkohol verwendet werden kann, oder mit einem Metallhydrid, wie Lithiumaluminiumhydrid, Diboran oder Natriumborhydrid, durchgeführt. Hierbei entstehen die Verbindungen der Formel I, in denen $R^2$ und $R^3$ Wasserstoffatome bedeuten.

Für die Umsetzung b) werden vorzugsweise Grignard-Verbindungen der Formel $R^2MgHal$ oder $R^2Li$ verwendet. Dabei entstehen die Verbindungen der Formel I, in denen $R^2$ ein Kohlenwasserstoffrest und $R^3$ ein Wasserstoffatom bedeutet. Diese Reaktion wird, wie bei Grignardreaktionen üblich, in Ether oder auch Tetrahydrofuran bei Temperaturen zwischen 0 und 60° durchgeführt, wobei die Grignard-Verbindung in einem Überschuß eingesetzt wird. Die Einwirkung kann sowohl auf die freien Basen als auch die Salze der Verbindungen der Formel II erfolgen.

Die so erhaltenen Verbindungen der Formel I, für die $R^3$ ein Wasserstoffatom bedeutet, können in bekannter Weise in ihre Alkyl- oder Acylderivate überführt werden. Umsetzung mit Acyl- oder Alkylhalogeniden in einem dipolar-aprotischen Lösungsmittel, wie vorzugsweise Dimethylformamid, in Gegenwart einer Base, wie Natriumhydrid, führt zu den gewünschten Verbindungen der Formel I, in denen $R^3$ die obengenannte Bedeutung mit Ausnahme von Wasserstoff hat. Die Carbonamido-Verbindungen ($R^3$ = CO—NH—$R^6$) erhält man aus den Verbindungen I ($R^3$ = OH) durch Umsetzung mit den entsprechenden Isocyanaten in einem Lösungsmittel wie Acetonitril.

Die Verbindungen I enthalten 2 asymmetrische Kohlenstoffatome, so daß mit Diastereomeren gerechnet werden mußte. Es zeigte sich jedoch, daß infolge asymmetrischer Induktion sowohl bei der Reduktion als auch bei der Einwirkung von metallorganischen Reagenzien auf die Verbindungen der Formel II nur ein Diastereomeres auftritt, welches gegebenenfalls in herkömmlicher Weise in die Enantiomeren aufgespalten werden kann.

Die zur Herstellung der Verbindungen I benötigten Ausgangsstoffe der Formel II sind noch nicht beschrieben. Sie lassen sich aus den entsprechenden β-Phenoxy- bzw. β-Thiophenoxypropiophenonen durch Umsetzung mit N,N-substituiertes Methylenimoniumchloriden darstellen (vgl. Angew. Chem. 88, 2161 (1976)).

Die erfindungsgemäßen Substanzen eignen sich zur Behandlung von psychischen Erkrankungen, insbesondere von Depressionen.

Als Wirkungsmechanismus einer Gruppe therapeutisch vielfach genutzter Antidepressiva (z. B. tricyclische Antidepressiva) ist eine Hemmung der neuronalen Aufnahme von Transmittersubstanzen (Noradrenalin und/oder Serotonin) anzusehen. Diese Eigenschaft wird in biologischen Testmodellen zur Charakterisierung von potentiellen Antidepressiva verwendet. Es wurde folgende Methodik eingesetzt :

Hemmung der Neurotransmitter-Aufnahme in Rattenhirn-Synaptosomen

Hippocampus und Cortex aus Rattenhirn wurden präpariert und in 0,32 M Sucrose-Lösung homogenisiert. Durch differentielle Zentrifugation wurden Synaptosomen erhalten, die in Pufferlösung suspendiert wurden. Die Synaptosomen sind in der Lage, aus dem umgebenden Medium zugefügte Neurotransmittersubstanzen (z. B. Noradrenalin oder Serotonin) aktiv aufzunehmen. Dieser Vorgang ist durch Aufnahme-Hemmer konzentrationsabhängig antagonisierbar. Die Synaptosomen wurden mit den Testsubstanzen in verschiedenen Konzentrationen versetzt und anschließend mit $^3$H-Noradrenalin (Hippocampus) bzw. $^3$H-Serotonin (Cortex) bei 37 °C inkubiert. Die Substratkonzentration betrug ca. 10 nM. Nach Stoppen der Aufnahme durch Verdünnen mit eisgekühlter Pufferlösung wurden die Synaptosomen abzentrifugiert und die $^3$H-Aktivität im Sediment gemessen. Der Blindwert wird durch Inkubation bei 0 °C ermittelt.

Aus den bei verschiedenen Inhibitor-Konzentrationen gegen Kontrolle ermittelten Hemmwerten wurde durch lineare Regression nach logit-log-Transformation die mittlere Hemmkonzentration ($IC_{50}$) berechnet.

In diesem Testmodell zeichnet sich das Standard-Antidepressivum Imipramin (siehe Tabelle 1) dadurch aus, daß es sowohl die Noradrenalin-Aufnahme, als auch die Serotonin-Aufnahme in niedrigem Konzentrationsbereich hemmt, wobei die Noradrenalin-Aufnahme-Hemmung deutlich im Vordergrund steht. Andererseits findet sich für das neu entwickelte Antidepressivum Femoxetin (siehe Tabelle 2) eine bevorzugte Hemmung der Serotonin-Aufnahme.

Die erfindungsgemäßen Substanzen lassen sich vom Wirkungstyp her in 3 verschiedene Gruppen einteilen. Eine erste Gruppe entspricht durch eine bevorzugte Hemmung der Noradrenalin-Aufnahme dem Imipramin-Typ, eine zweite entspricht durch bevorzugte Hemmung der Serotonin-Aufnahme dem Femoxetin-Typ und eine dritte Gruppe zeichnet sich durch eine etwa gleich starke Aufnahme-Hemmung beider Neurotransmiter aus.

Die Substanzen der Gruppe 1 sind in Tabelle 1 zusammengestellt. Es finden sich hier Substanzen, die das Imipramin in der absoluten Wirkstärke bei der Hemmung der Noradrenalin-Aufnahme zum Teil sehr deutlich (8-fach bei Beispiel 1.6 und 5) übertreffen. Auch die Serotonin-Aufnahme wird, wenn auch in deutlich höheren Konzentrationen, gehemmt. Ausdruck dieser spezifischen Noradrenalin-Aufnahme-Hemmung ist der Quotient in der letzten Spalte der Tabelle. Je höher dieser Wert ist, desto spezifischer ist die Noradrenalin-Aufnahme-Hemmung. Alle aufgreführten Quotienten liegen, zum Teil sehr deutlich, über dem Quotienten des Imipramin.

Tabelle 2 zeigt die erfindungsgemäßen Verbindungen, bei denen die Hemmung der Serotonin-Aufnahme im Vordergrund steht. Alle aufgeführten Beispiele sind (bis maximal 12-fach) wirksamer als die Vergleichssubstanz Femoxetin. Auch die Noradrenalin-Aufnahme wird, wenn auch durch deutlich höhere Konzentrationen, gehemmt. Der Quotient der letzten Spalte ist Ausdurck der Spezifität der Serotonin-Aufnahme-Hemmung, wobei ein größerer Quotient einer größeren Spezifität entspricht. 3 der in der Tabelle aufgeführten Beispiele zeigen somit eine deutlich höhere Spezifität als das Femoxetin.

Die Substanzen der 3. Gruppe zeichnen sich durch eine gleich starke Hemmung der Aufnahme beider Transmitter aus. Die Ergebnisse sind in Tabelle 3 zusammengestellt. Die IC 50-Werte für die Aufnahme-Hemmung von Noradrenalin sowie Serotonin liegen jeweils in einem niedrigen Konzentrationbereich. Der Quotient beider IC 50-Werte liegt jedoch überwiegend in der Nähe von 1 als Ausdruck einer gleichförmigen Hemmung beider Transmitter. Dadurch unterscheiden sich die Substanzen einerseits deutlich von Imipramin, bei dem die Noradrenalin-Aufnahme-Hemmung mit einem Faktor von 9,3 deutlich

im Vordergrund steht, zum anderen auch deutlich von Femoxetin, bei dem die Serotonin-Aufnahme-Hemmung die des Noradrenalins um den Faktor 10 übertrifft.

Nach einer allgemein anerkannten Arbeitshypothese wird als Ursache von Depressionen eine Störung im Monoaminstoffwechsel angenommen. Neuere Untersuchungen zeigen eine Differenzierung in verschiedene Depressionsformen, wobei einmal eine Störung des Noradrenalin-Stoffwechsels, zum anderen eine Störung des Serotonin-Stoffwechsels im Vordergrund stehen kann. In der Mehrzahl der Fälle sind wahrscheinlich beide Transmittersysteme in Mitleidenschaft gezogen.

Entsprechend diesen klinischen Vorstellungen können die erfindungsgemäßen Substanzen bei der Behandlung derartiger depressiver Störungen eingesetzt werden. So erscheinen die Substanzen der Gruppe 1 bei Störungen im Noradrenalin-Haushalt angezeigt, die Substanzen der Gruppe 2 bei Störungen im Serotonin-Haushalt und die Substanzen der Gruppe 3 bei den erwähnten Mischformen.

Tabelle 1

| Substanz des Beispiels Nr. | Hemmung der Neurotransmitter-Aufnahme in Synaptosomen | | |
|---|---|---|---|
| | Noradrenalin $IC_{50}$ (µmol/l) | Serotonin $IC_{50}$ (µmol/l) | Quotient $\frac{IC_{50}\ \text{Serotonin}}{IC_{50}\ \text{Noradrenalin}}$ |
| 1 | 0,0052 | 0,19 | 36,5 |
| 1.3 | 0,32 | 0,49 | 15,3 |
| 1.6 | 0,0017 | 0,065 | 38,2 |
| 5 | 0,0017 | 0,40 | 235,0 |
| 5.3 | 0,020 | 0,30 | 15,0 |
| Imipramin | 0,014 | 0,13 | 9,3 |

Tabelle 2

| Substanz des Beispiels Nr. | Hemmung der Neurotransmitter-Aufnahme in Synaptosomen | | |
|---|---|---|---|
| | Serotonin $IC_{50}$ (µmol/l) | Noradrenalin $IC_{50}$ (µmol/l) | Quotient $\frac{IC_{50}\ \text{Noradrenalin}}{IC_{50}\ \text{Serotonin}}$ |
| 1.2 | 0,020 | 0,18 | 9,0 |
| 2.6 | 0,0036 | 0,20 | 55,6 |
| 2.7 | 0,0031 | 0,15 | 48,4 |
| 2.8 | 0,0038 | 0,16 | 42,1 |
| Femoxetin | 0,036 | 0,33 | 9,2 |

(Siehe Tabelle 3 Seite 5 f.)

Tabelle 3

| Substanz des Beispiels Nr. | Hemmung der Neurotransmitter-Aufnahme in Synaptosomen | | |
|---|---|---|---|
| | Noradrenalin $IC_{50}$ (µmol/l) | Serotonin $IC_{50}$ (µmol/l) | Quotient $IC_{50}$ Serotonin / $IC_{50}$ Noradrenalin |
| 1.4 | 0,017 | 0,073 | 4,3 |
| 2 | 0,011 | 0,025 | 2,3 |
| 2.2 | 0,036 | 0,066 | 1,8 |
| 2.3 | 0,013 | 0,037 | 2,8 |
| 2.7 | 0,038 | 0,049 | 1,3 |
| 2.8 | 0,50 | 0,30 | 0,6 |
| 2.9 | 0,10 | 0,069 | 0,7 |
| 6 | 0,017 | 0,056 | 3,3 |
| Imipramin | 0,014 | 0,13 | 9,3 |
| Femoxetin | 0,33 | 0,036 | 0,11 |

Gegenstand der Erfindung sind daher auch Arzneimittel, die eine Verbindung der Formel I enthalten, sowie deren Verwendung bei der Bekämpfung von Krankheiten.

Die erfindungsgemäßen Verbindungen konnen in üblicher Weise oral oder parenteral verabfolgt werden.

Die Dosierung hängt vom Alter, Zustand und Gewicht des Patienten sowie von der Applikationsart ab. In der Regel beträgt die tägliche Wirkstoffdosis zwischen etwa 0,1 und 10 mg/kg Körpergewicht.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien, Lösungen, Salben, Cremes oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmittel, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker et al : Pharmazeutische Technologie, Thieme-Verlag, Stuttgart, 1978). Die so erhaltenen Applikationsformen enthalten den Wirkstoff normalerweise in einer Menge von 1 bis 99 Gew.%.

Die neuen Substanzen können auch in Form ihrer Salze mit physiologisch verträglichen Säuren verabfolgt werden. Solche Säuren sind beispielsweise Salzsäure, Schwefelsäure, Phosphorsäure, Weinsäure, Essigsäure, Bernsteinsäure, Milchsäure, Zitronensäure, Amidosulfonsäure.

Die folgenden Beispiele erläutern die Erfindung :

## A. Herstellung der Ausgangsstoffe

a) 3-Dimethylamino-2-phenoxymethyl-1-phenylpropanon-(1)-hydrochlorid

22,6 g (0,1 Mol) β-Phenoxypropiophenon (hergestellt durch Umsetzung von β-Chlorpropiophenon mit Phenol in alkalischer Lösung) wurden mit 10 g N,N-Dimethylmethylenimoniumchlorid (hergestellt aus Bis-dimethylaminomethan und Acetylchlorid) in 200 ml trockenem Acetonitril 3 Stunden unter Rühren auf 65 °C erwärmt. Die Reaktionsmischung wurde sodann in 1 1 Ether gegossen, der Niederschlag abgesaugt und in dieser Form für die weiteren Umsetzungen verwendet. Ausbeute : 75 %, Fp. 128 bis 132 °C.

Unter Verwendung substituierter Phenole bzw. Thiophenol wurden analog erhalten :

b) 1-(4-Chlorphenyl)-3-dimethylamino-2-phenoxymethyl-propanon-(1) Hydrochlorid, Fp. 170 bis 173 °C.

c) 3-Dimethylamino-1-(4-fluorphenyl)-2-phenoxymethyl-propanon-(1) Hydrochlorid, Fp. 135 bis 137 °C.

d) 2-(4-Chlorphenoxymethyl)-3-dimethylamino-phenyl-propanon-(1) Hydrochlorid, Fp. 153 bis 155 °C.

e) 2-(4-Bromphenoxymethyl)-3-dimethylamino-1-phenyl-propanon-(1) Hydrochlorid, Fp. 150 bis 152 °C.

f) 3-Dimethylamino-1-phenyl-2-thiophenoxymethyl-propanon-(1) Hydrochlorid, Fp. 136 bis 138 °C.

g) 2-(2-Chlorphenoxymethyl)-3-dimethylamino-1-phenyl-propanon-(1) Hydrochlorid, Fp. 138 bis 140 °C.

h) 2-(4-Chlorthiophenoxymethyl)-3-dimethyl-amino-1-phenyl-propanon-(1) Hydrochlorid Fp. 156 bis 158 °C.

Unter Verwendung von Piperidin- und Pyrrolidinmethylenimoniumchlorid (hergestellt aus Dipiperidinomethan bzw. Dipyrrolidinomethan und Acetylchlorid) wurden erhalten :

i) 1-Phenyl-3-piperidinyl-2-thiophenoxymethyl-propanon-(1) Hydrochlorid Fp. 161 bis 163 °C.

k) 2-Phenoxymethyl-1-phenyl-3-piperidinyl-propanon-(1) Hydrochlorid, Fp. 118 bis 121 °C.

l) 2-Phenoxymethyl-1-phenyl-3-pyrrolidinylpropanon-(1) Hydrochlorid, ölige unreine Substanz.

## B. Herstellung der Endprodukte

### Beispiel 1

46 g (0,17 Mol) 3-Dimethylamino-2-phenoxymethyl-1-phenylpropanon-(1) (freigesetzt aus dem nach A.a) erhaltenen Hydrochlorid) wurden in 200 ml absolutem Tetrahydrofuran gelöst und im Verlauf von 45 min in der Siedehitze zu einer Suspension von 10 g Lithiumaluminiumhydrid in 100 ml absolutem Tetrahydrofuran getropft. Nach 2 Stunden Kochen unter Rühren und Rückfluß wurde mit Wasser und verdünnter Natronlauge zersetzt, abgesaugt und der eingedampfte Rückstand aus 2 Teilen Isopropanol umkristallisiert. Man erhielt 20,3 g (45 %) 1-Dimethylamino-2-phenoxymethyl-3-phenyl-propanol-(3) (1.1), Fp. 107 bis 109 °C.

Ebenso wurden erhalten :

1.2) 2-(4-Chlorphenoxymethyl)-1-dimethylamino-3-phenyl-propanol-(3), Fp. 98 bis 100 °C.

1.3) 1-Dimethylamino-3-(4-fluorphenyl)-2-phenoxymethyl-propanol-(3) Hydrochlorid, Fp. 146 bis 150 °C.

1.4) 2-(4-Bromphenoxymethyl)-1-dimethylamino-3-phenyl-propanol-(3) Hydrochlorid, Fp. 199 bis 200 °C.

1.5) 3-(4-Chlorphenyl)-1-dimethylamino-2-phenoxymethyl-propanol-(3) Hydrochlorid, Fp. 170 bis 173 °C.

1.6) 1-Dimethylamino-3-phenyl-2-thiophenoxymethyl-propanol-(3), Fp. 88 bis 90 °C.

1.7) 2-(2-Chlorphenoxymethyl)-1-dimethylamino-3-phenyl-propanol-(3) Hydrochlorid, Fp 188 bis 190 °C.

1.8) 2-(4-Chlorthiophenoxymethyl)-1-dimethylamino-3-phenyl-propanol-(3), Fp 108 bis 110 °C.

1.9) 3-Phenyl-1-piperidinyl-2-thiophenoxymethyl-propanol-(3) Hydrochlorid, Fp 201 bis 203 °C.

1.10) 2-Phenoxymethyl-3-phenyl-1-piperidinyl-propanol-(3) Hydrochlorid, Fp 190 bis 192 °C.

1.11) 2-Phenoxymethyl-3-phenyl-1-pyrrolidinyl-propanol-(3) Fp 100 bis 103 °C.

### Beispiel 2

Zu der aus 20,5 g (0,188 Mol) Ethylbromid und 4,5 g Magnesium in 300 ml Ether hergestellten Grignard-Lösung wurde portionsweise 20 g (0,063 Mol) von den nach Beispiel a) erhaltenen 3-Dimethylamino-2-phenoxymethyl-1-phenylpropanon-(1) Hydrochlorid gegeben und 5 h bei Raumtemperatur gerührt. Anschließend wurd mit gesättigter Ammoniumchloridlösung zersetzt, die Lösung getrocknet und durch Einleiten von Chlorwasserstoffgas 1-Dimethylamino-3-phenyl-2-phenoxymethyl-pentanol-(3) Hydrochlorid (2.1) gefällt, welches aus Isopropanol umkristallisiert wurde. Ausbeute : 12 g (55 %), Fp. 211 bis 212 °C.

Ebenso wurden hergestellt :

2.2) 1-Dimethylamino-2-phenoxymethyl-3-phenyl-butanol-(3) Hydrochlorid, Fp. 188 bis 190 °C.

2.3) 1-Dimethylamino-2-phenoxymethyl-3-phenyl-hexanol-(3) Hydrochlorid, Fp. 183 bis 185 °C.

2.4) 2-(4-Chlorphenoxymethyl)-1-dimethylamino-3-phenyl-hexanol-(3) Hydrochlorid, Fp. 159 bis 161 °C.

2.5) 2-(4-Chlorphenoxymethyl)-1-dimethylamino-3-phenyl-pentanol-(3) Hydrochlorid, Fp. 225 °C.

2.6) 2-(4-Chlorphenoxymethyl)-1-dimethylamino-3-phenyl-butanol-(3) Hydrochlorid, Fp. 194 bis 196 °C.

2.7) 1-Dimethylamino-2-phenoxymethyl-3-phenyl-undecanol-(3) (chromatographisch an Kieselgel/Methylenchlorid gereinigt),

2.8) 1-Dimethylamino-2-phenoxymethyl-3-phenyl-pentadecanol-(3), Fp. 54 bis 57 °C.

2.9) 1-Dimethylamino-2-phenoxymethyl-3-phenyl-pent-4-enol-(3) Hydrochlorid, Fp. 167 bis 169 °C.

2.10) 3-Cyclopropyl-1-dimethylamino-2-phenoxymethyl-3-phenyl-propanol-(3) Hydrochlorid, Fp. 185 bis 188 °C.

2.11) 1-Dimethylamino-3-phenyl-2-(thiophenoxy-methyl)-pentanol-(3) Hydrochlorid, Fp. 207 bis 210 °C.

2.12) 2-Phenoxymethyl-3-phenyl-1-piperidinyl-pentanol-(3) Hydrochlorid, Fp. 206 bis 208 °C.

## Beispiel 3

Ersetzt man in Beispiel 2 die Grignard-Verbindung durch 0,188 Mol Lithiumacetylid-Ethylendiamin-Komplex in 300 ml Tetrahydrofuran und das 3-Dimethylamino-2-phenoxymethyl-1-phenyl-propdanon-(1)-hydrochlorid durch die freie Base, so erhält man analog Beispiel 2 nach Chromatographie an Kieselgel/Methylenchlorid 11 g (50 %) 1-Dimethylamino-2-phenoxymethyl-3-phenyl-pent-4-in-ol-(3) Oxalat, Fp. 123 bis 125 °C.

## Beispiel 4

10,7 g (0,037 Mol) 1-Dimethylamino-2-phenoxy-3-phenyl-methyl-pentanol-(3) (erhalten nach Beispiel 2) wurden mit 14,06 g (0,037 Mol) L-(—)-Dibenzoylweinsäuremonohydrat in 75 ml Isopropanol gelöst und 400 ml Ether zugefügt. Nach einiger Zeit fällt ein Kristallisat aus, das nach zweimaligem Umkristallisieren einen konstanten Drehwert von $[\alpha]_D^{20} = -69{,}8°$ (Ethanol, 10 mg/ml) aufwies. Die daraus freigesetzte linksdrehende Base (4.1) ergab einen Drehwert von $[\alpha]_D^{20} = -27{,}1°$, das Hydrochlorid $[\alpha]_D^{20} = -15{,}5°$. Der Schmelzpunkt betrug 226 bis 227 °C.

4.2) Der optische Antipode wurde mit D-(+)-Dibenzoylweinsäure auf analoge Weise erhalten : $[\alpha]_D^{20} = +26{,}6°$, Hydrochlorid : $[\alpha]_D^{20} = +15{,}9°$, Fp. 226 bis 227 °C. Alle Drehwerte wurden in Ethanol bei einer Konzentration von c = 10 mg/ml gemessen.

## Beispiel 5

In eine Lösung von 0,8 g (0,033 Mol) Natriumhydrid in 50 ml absolutem Dimethylformamid wurde bei Raumtemperatur eine Lösung von 8,5 g (0,03 Mol) 1-Dimethylamino-2-phenoxymethyl-3-phenyl-propanol-(3) (nach Beispiel 1 erhalten) in 25 ml Dimethylformamid langsam eingetropft. Anschließend fügte man tropfenweise unter Temperaturanstieg auf 40 °C 6 g (0,06 Mol) Acetanhydrid in 25 ml Dimethylformamid zu und rührte noch 2 h bei Raumtemperatur.

Der Kolbeninhalt wurde in 300 ml Wasser gegeben, 3 x mit je 100 ml Ether extrahiert und aus der Etherlösung durch Einleiten von Chlorwasserstoffgas das 3-Acetoxy-1-dimethylamino-2-phenoxymethyl-3-phenylpropan Hydrochlorid (5.1) gefällt, welches anschließend aus Isopropanol umkristallisiert wurde. Ausbeute : 7,1 g (65 %), Fp. 215 bis 217 °C.

Analog wurden erhalten :

5.2) 1-Dimethylamino-3-ethoxycarbonyloxy-2-phenoxymethyl-3-phenyl-propan Hydrochlorid, Fp. 183 bis 186 °C.

5.3) 3-Acetoxy-1-dimethylamino-2-phenoxymethyl-3-phenyl-pentan Hydrochlorid, Fp. 173 bis 174 °C.

5.4) 3-Acetoxy-1-dimethylamino-3-phenyl-2-thiophenoxymethyl-propan Hydrochlorid, Fp. 185 bis 187 °C.

5.5) 3-Acetoxy-2-phenoxymethyl-3-phenyl-1-piperidinyl-propan, Hydrochlorid, Fp. 80 bis 82 °C.

## Beispiel 6

Nach dem Verfahren von Beispiel 5 wurde durch Austausch des Acetanhydrids gegen die äquimolekulare Menge Allylbromid 3-Allyloxy-1-dimethylamino-2-phenoxymethyl-3-phenyl-propan (6.1) erhalten. Ausbeute : 11,8 g (95 %) Oxalat : Fp. 140 bis 143 °C.

Ebenso wurden hergestellt :

6.2) 1-Dimethylamino-3-methoxy-2-phenoxymethyl-3-phenyl-propan Oxalat, Fp. 145 bis 148 °C.

## Beispiel 7

1,4 g (0,005 Mol) des nach Beispiel 1.1 erhaltenen 1-Dimethylamino-2-phenoxymethyl-3-phenyl-propanols-(3) wurden mit 1,0 g Butylisocyanat (0,01 Mol) in 25 ml Acetonitril 3 h unter Rückfluß erhitzt. Das nach Abdestillieren des Lösungsmittels erhaltene Öl wurde mit etherischer Salzsäure in das Hydrochlorid überführt. Man erhielt 1,0 g (48 %) 3-(Butylaminocarbonyloxy)-1-dimethylamino-2-phenoxymethyl-3-phenyl-propan Hydrochlorid (7.1), Fp. 137 bis 140 °C.

Ebenso wurden erhalten :

7.2) 1-Dimethylamino-3-(ethylamino-carbonyloxy)-2-phenoxymethyl-3-phenyl-propan Hydrochlorid, Fp. 169 bis 171 °C.

## Beispiel I

Auf einer Tablettenpresse wurden in üblicher Weise Tabletten folgender Zusammensetzung gepreßt :

10,00 mg 1-Dimethylamino-2-phenoxymethyl-3-phenyl-pentanol-(3)-hydrochlorid
50,00 mg Maisstärke
4,50 mg Gelatine
15,00 mg Milchzucker
7,50 mg Talk
0,75 mg Aerosil(R) (chemisch reine Kieselsäure in submikroskopisch feiner Verteilung)
2,25 mg Kartoffelstärke (als 6 %iger Kleister)

Beispiel II

In üblicher Weise wurden Dragees folgender Zusammensetzung hergestellt :

10,00 mg 1-Dimethylamino-2-phenoxymethyl-3-phenyl-pentanol-(3)-hydrochlorid
50,00 mg Kernmasse
40,00 mg Verzuckerungsmasse

Die Kernmasse besteht aus : 9 Teilen Maisstärke, 3 Teilen Milchzucker und 1 Teil Luviskol(R) VA 64 (Vinylpyrrolidon-Vinylacetat-Mischpolymerisat 60 : 40, vgl. Pharm. Ind. *1962,* 586).

Die Verzuckerungsmasse besteht aus 5 Teilen Rohrzucker, 2 Teilen Maisstärke, 2 Teilen Calciumcarbonat und 1 Teil Talk. Die so hergestellten Dragees wurden anschließend mit einem magensaftresistenten Überzug versehen.

Beispiel III

Es wurden 5 g 1-Dimethylamino-2-phenoxymethyl-3-phenyl-pentanol-(3)-hydrochlorid in 2,01 Wasser gelöst, auf pH 6 eingestellt, mit Kochsalz isotonisch eingestellt und in 2 ml fassende Ampullen steril abgefüllt.

**Patentansprüche** (für die Vertragsstaaten : BE, CH, DE, FR, GB, IT, LI, NL, SE)

1. Phenylpropanolamine der Formel I

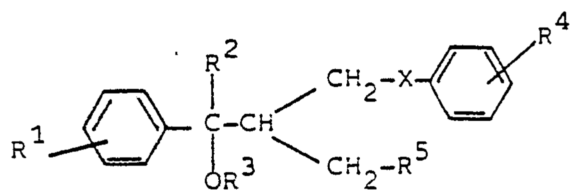

(I)

worin

$R^1$ ein Wasserstoff- oder ein Halogenatom,

$R^2$ ein Wasserstoffatom, einen gesättigten oder ungesättigten Kohlenwasserstoffrest mit bis zu 15 Kohlenstoffatomen

$R^3$ ein Wasserstoffatom, einen gesättigten oder ungesättigten Kohlenwasserstoffrest mit bis zu 3 Kohlenstoffatomen, eine $C_{1-4}$-Acyl-, Benzoyl-, $C_{2-4}$-Carbalkoxygruppe oder eine Carbonamidogruppe -CO-NHR$^6$, worin $R^6$ eine $C_{1-4}$-Alkylgruppe darstellt,

$R^4$ ein Wasserstoff- oder Halogenatom,

$R^5$ eine Dimethylamino-, Piperidino- oder Pyrrolidinogruppe und

X ein Sauerstoff- oder Schwefelatom

bedeuten, sowie deren Salze mit physiologisch verträglichen Säuren.

2. 1-Dimethylamino-2-phenoxymethyl-3-phenyl-pentanol-(3).

3. 1-Dimethylamino-2-phenoxymethyl-3-phenyl-propanol-(3).

4. 3-Acetoxy-1-dimethylamino-2-phenoxymethyl-3-phenyl-propan.

5. 1-Dimethylamino-3-phenyl-2-thiophenoxymethyl-propanol-(3).

6. Verfahren zur Herstellung von Phenylpropanolaminen der Formel I gemäß Anspruch 1, dadurch gekennzeichnet, daß man eine Verbindung der Formel II

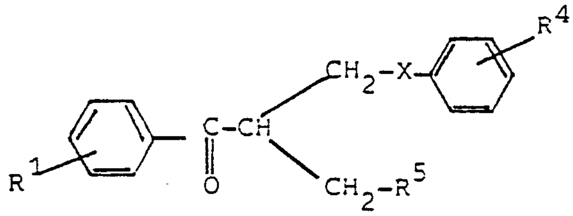

(II)

worin $R^1$, $R^4$, $R^5$ und X die vorstehend genannte Bedeutung haben,

a) reduziert oder

b) mit einer entsprechend substituierten metallorganischen Verbindung umsetzt

und in die so erhaltenen Verbindungen gegebenenfalls verethert oder verestert und/oder in ihre Salze mit physiologisch verträglichen Säuren überführt.

7. Arzneimittel, enthaltend eine Verbindung gemäß Anspruch 1.

8. Verbindung gemäß Anspruch 1 zur Verwendung bei der Bekämpfung von Krankheiten.

**Patentansprüche** (für den Vertragsstaat AT)

1. Verfahren zur Herstellung von Phenylpropanolaminen der Formel I

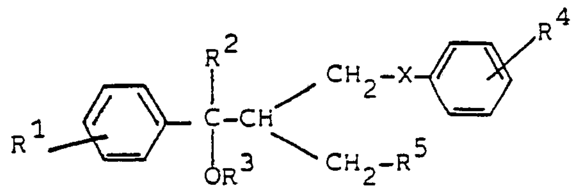

(I)

worin

$R^1$ ein Wasserstoff- oder ein Halogenatom,

$R^2$ ein Wasserstoffatom, einen gesättigten oder ungesättigten Kohlenwasserstoffrest mit bis zu 15 Kohlenstoffatomen,

$R^3$ ein Wasserstoffatom, einen gesättigten oder ungesättigten Kohlenwasserstoffrest mit bis zu 3 Kohlenstoffatomen, eine $C_{1-4}$-Acyl-, Benzoyl-, $C_{2-4}$-Carbalkoxygruppe oder eine Carbonamidogruppe —CO—NHR$^6$, worin $R^6$ eine $C_{1-4}$-Alkylgruppe darstellt,

$R^4$ ein Wasserstoff- oder Halogenatom,

$R^5$ eine Dimethylamino-, Piperidino- oder Pyrrolidinogruppe und

X ein Sauerstoff- oder Schwefelatom

bedeuten, sowie deren Salzen mit physiologisch verträglichen Säuren, dadurch gekennzeichnet, daß man eine Verbindung der Formel II

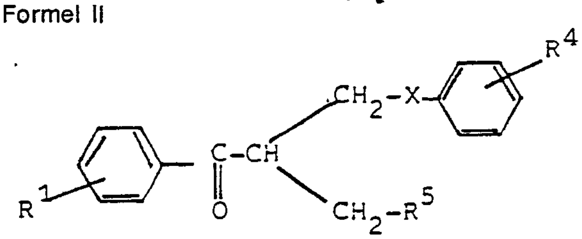

(II)

worin $R^1$, $R^4$, $R^5$ und X die vorstehend genannte Bedeutung haben,

a) reduziert oder

b) mit einer entsprechend substituierten metallorganischen Verbindung umsetzt

und in die so erhaltenen Verbindungen gegebenenfalls verethert oder verestert und/oder in ihre Salze mit physiologisch verträglichen Säuren überführt.

2. Verfahren gemäß Anspruch 1, dadurch gekennzeichnet, daß man 1-Dimethylamino-2-phenoxymethyl-3-phenyl-pentanol-(3) herstellt.

3. Verfahren gemäß Anspruch 1, dadurch gekennzeichnet, daß man 1-Dimethylamino-2-phenoxymethyl-3-phenyl-propanol-(3) herstellt.

4. Verfahren gemäß Anspruch 1, dadurch gekennzeichnet, daß man 3-Acetoxy-1-dimethylamino-2-phenoxymethyl-3-phenyl-propan herstellt.

5. Verfahren gemäß Anspruch 1, dadurch gekennzeichnet, daß man 1-Dimethylamino-3-phenyl-2-thiophenoxymethyl-propanol-(3) herstellt.

**Claims** (for the Contracting States : BE, CH, DE, FR, GB, IT, LI, NL, SE)

1. A phenylpropanolamine of the formula I

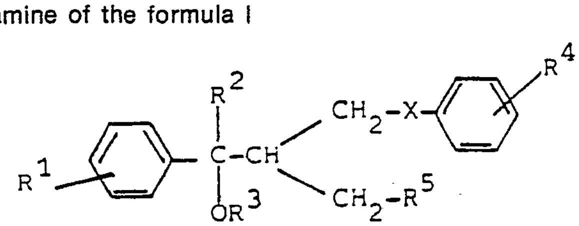

(I)

where

$R^1$ is hydrogen or halogen,

$R^2$ is hydrogen, or a saturated or unsaturated hydrocarbon radical of up to 15 carbon atoms,

$R^3$ is hydrogen, a saturated or unsaturated hydrocarbon radical of up to 3 carbon atoms, $C_1$-$C_4$-acyl, benzoyl, $C_2$-$C_4$-carbalkoxy or a carboxamido group —CO—$NHR^6$, where $R^6$ is $C_1$-$C_4$-alkyl,

$R^4$ is hydrogen or halogen,

$R^5$ is dimethylamino, piperidino or pyrrolidono, and X is oxygen or sulfur,

and its salts with physiologically tolerated acids.

2. 1-Dimethylamino-2-phenoxymethyl-3-phenylpentan-3-ol.

3. 1-Dimethylamino-2-phenoxymethyl-3-phenylpropan-3-ol.

4. 3-Acetoxy-1-dimethylamino-2-phenoxymethyl-3-phenylpropane.

5. 1-Dimethylamino-3-phenyl-2-thiophenoxymethylpropan-3-ol.

6. A process for the preparation of a phenylpropanalamine of the formula I as claimed in claim 1, wherein a compound of the formula II

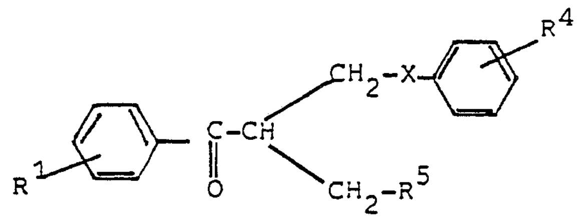

(II)

where $R^1$, $R^4$, $R^5$ and X have the above meanings,

(a) is reduced, or

(b) is reacted with an appropriately substituted organometallic compound,

and, if desired, the compound thus obtained is etherified or esterified and/or converted into its salts with physiologically tolerated acids.

7. A pharmaceutical containing a compound as claimed in claim 1.

8. A compound as claimed in claim 1 for use in the treatment of disorders.

**Claims** (for the Contracting State AT)

1. A process for the preparation of a phenylpropanolamine of the formula I

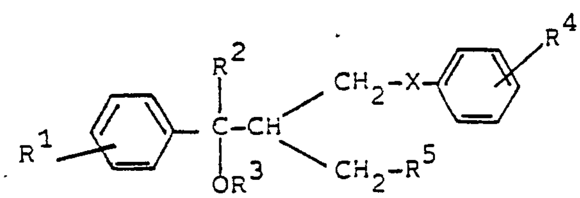

(I)

where

$R^1$ is hydrogen or halogen,

$R^2$ is hydrogen, or a saturated or unsaturated hydrocarbon radical of up to 15 carbon atoms,

$R^3$ is hydrogen, a saturated or unsaturated hydrocarbon radical of up to 3 carbon atoms, $C_1$-$C_4$-acyl, benzoyl, $C_2$-$C_4$-carbalkoxy or a carboxamido group —CO—$NHR^6$, where $R^6$ is $C_1$-$C_4$-alkyl,

$R^4$ is hydrogen or halogen,

$R^5$ is dimethylamino, piperidino or pyrrolidino, and

X is oxygen or sulfur,

and its salts with physiologically tolerated acids, wherein a compound of the formula II

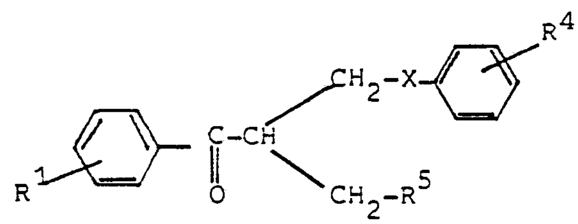

(II)

where $R^1$, $R^4$, $R^5$ and X have the above meanings,

(a) is reduced, or

(b) is reacted with an appropriately substituted organometallic compound,

and, if desired, the compound thus obtained is etherified or esterified and/or converted into its salts with physiologically tolerated acids.

2. A process as claimed in claim 1, wherein 1-dimethylamino-2-phenoxymethyl-3-phenylpentan-3-ol is prepared.

3. A process as claimed in claim 1, wherein 1-dimethylamino-2-phenoxymethyl-3-phenylpropan-3-ol is prepared.

4. A process as claimed in claim 1, wherein 3-acetoxy-1-dimethylamino-2-phenoxymethyl-3-phenylpropane is prepared.

5. A process as claimed in claim 1, wherein 1-dimethylamino-3-phenyl-2-thiophenoxymethylpropan-3-ol is prepared.

**Revendications** (pour les Etats contractants : BE, CH, DE, FR, GB, IT, LI, NL, SE)

1. Phénylpropanolamines de formule I

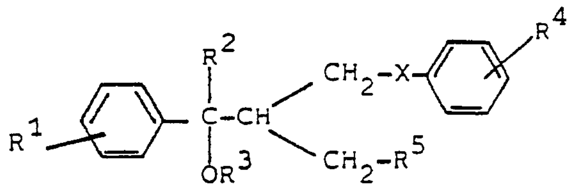

(I)

dans laquelle

$R^1$ représente un atome d'hydrogène ou un atome d'halogène,

$R^2$, un atome d'hydrogène, un reste d'hydrocarbure saturé ou non saturé ayant jusqu'à 15 atomes de carbone,

$R^3$, un atome d'hydrogène, un reste d'hydrocarbure saturé ou non saturé ayant jusqu'à 3 atomes de carbone, un groupe acyle en $C_{1-4}$, carbalcoxy en $C_{2-4}$, ou un groupe carbonamide —CO—NHR$^6$ où $R^6$ représente un groupe alkyle en $C_{1-4}$,

$R^4$ représente un atome d'hydrogène ou d'halogène,

$R^5$ un groupe diméthylamino, pipéridino ou pyrrolidino et

X un atome d'oxygène ou de soufre

ainsi que leurs sels d'acides acceptables physiologiquement.

2. 1-Diméthylamino-2-phénoxyméthyl-3-phényl-pentanol-(3).

3. 1-Diméthylamino-2-phénoxyméthyl-3-phényl-propanol-(3).

4. 3-Acétoxy-1-diméthylamino-2-phénoxyméthyl-3-phényl-propane.

5. 1-Diméthylamino-3-phényl-2-thiophénoxyméthyl-propanol-(3).

6. Procédé de préparation de phénylpropanolamines de formule I selon la revendication 1, caractérisé par le fait que :

a) on réduit ou

b) on fait réagir avec un composé organométallique substitué correspondant

un composé de formule II

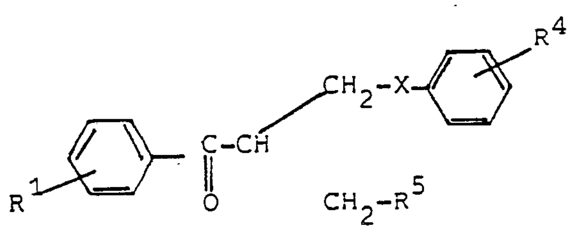

(II)

et on transforme en les composés ainsi obtenus éventuellement éthérifiés ou estérifiés et/ou en leurs sels d'acides acceptables physiologiquement.

7. Médicament, contenant un composé selon la revendication 1.

8. Composé selon la revendication 1 pour l'utilisation pour la lutte contre des maladies.

**Revendications** (pour l'Etat contractant AT)

1. Procédé de préparation de phénylpropanolamines de formule I

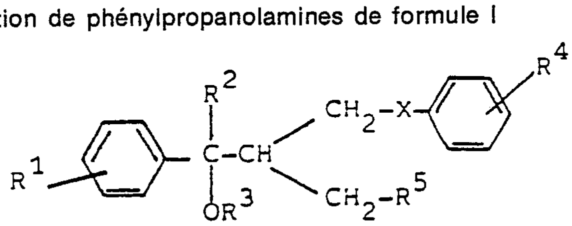

(I)

dans laquelle

$R^1$ représente un atome d'hydrogène ou un atome d'halogène,

$R^2$, un atome d'hydrogène, un reste d'hydrocarbure saturé ou non saturé ayant jusqu'à 15 atomes de carbone

$R^3$, un atome d'hydrogène, un reste d'hydrocarbure saturé ou non saturé ayant jusqu'à 3 atomes de carbone, un groupe acyle en $C_{1-4}$, carbalcoxy en $C_{2-4}$, ou un groupe carbonamide —CO—NHR$^6$ où $R^6$ représente un groupe alkyle en $C_{1-4}$

$R^4$ représente un atome d'hydrogène ou d'halogène,

$R^5$ un groupe diméthylamino, pipéridino ou pyrrolidino et

X un atome d'oxygène ou de soufre

ainsi que leurs sels d'acides acceptables physiologiquement, caractérisé par le fait

a) qu'on réduit un composé de formule II

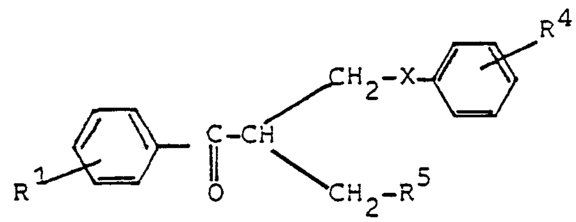

(II)

dans laquelle $R^1$, $R^4$, $R^5$ et X ont la signification ci-dessus, ou

b) qu'on fait réagir ce composé de formule II avec un composé métallo-organique substitué adéquatement et qu'on transforme les composés éthérifiés ou estérifiés ainsi obtenus et/ou en leurs sels d'acides acceptables physiologiquement.

2. Procédé selon la revendication 1, caractérisé par le fait que l'on prépare du 1-diméthylamino-2-phénoxyméthyl-3-phényl-pentanol-(3).

3. Procédé selon la revendication 1, caractérisé par le fait que l'on prépare du 1-diméthylamino-2-phénoxyméthyl-3-phényl-propanol-(3).

4. Procédé selon la revendication 1, caractérisé par le fait que l'on prépare du 3-acétoxy-1-diméthylamino-2-phénoxyméthyl-3-phényl-propane.

5. Procédé selon la revendication 1, caractérisé par le fait que l'on prépare du 1-diméthylamino-3-phényl-2-thiophénoxyméthyl-propanol-(3).